(19) Europäisches Patentamt
European Patent Office
Office européen des brevets

(11) Publication number: **0 146 886**
**B1**

# (12) EUROPEAN PATENT SPECIFICATION

(45) Date of publication of patent specification: **28.09.88**

(51) Int. Cl.$^4$: **C 07 C 43/11** // B01D53/34

(21) Application number: **84115342.2**

(22) Date of filing: **13.12.84**

## (54) Solvent composition for removing acidic gas components from gas mixtures.

(30) Priority: **19.12.83 US 562898**

(43) Date of publication of application:
**03.07.85 Bulletin 85/27**

(45) Publication of the grant of the patent:
**28.09.88 Bulletin 88/39**

(84) Designated Contracting States:
**BE DE FR GB NL**

(56) References cited:
**FR-A-2 045 972**
**FR-A-2 278 666**

(73) Proprietor: **NORTON COMPANY**
**1 New Bond Street**
**Worcester Massachusetts 01606 (US)**

(72) Inventor: **Kutsher, George, S.**
**76 Woodland Road**
**Dover, New Jersey 07801 (US)**
Inventor: **Valentine, John, P.**
**R.R. No.1, Box 235,**
**Belle Mead, New Jersey 08502 (US)**

(74) Representative: **Baillie, Iain Cameron et al**
**c/o Ladas & Parry Isartorplatz 5**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 146 886 B1

Courier Press, Leamington Spa, England.

## Description

The present invention relates to a mixture of dialkyl ethers of polyalkylene glycols useful in removing acidic gas components such as hydrogen sulfide from gas mixtures. Still more particularly the present invention relates to a mixture of dimethyl ethers of polyethylene glycol, having an average molecular weight of from about 200 to 255, and preferably from 227 to 244, which is effective at low temperatures for the removal of acidic gas components from gas mixtures.

U.S. Patent 3,737,392 teaches that a certain mixture of dimethyl ethers of polyethylene glycols is useful in treating and separating acid gas particularly hydrogen sulfide from gas mixtures such as natural gas mixtures containing hydrogen sulfide, carbon dioxide and methane. Such a solvent system available under the trademark SELEXOL has been widely and successfully used in many natural gas treating operations. Example 6 of FR—A—2 278 666 also teaches a mixture of oligomers which is probably suitable for the same purpose.

However, it has been found that such a solvent system does not operate efficiently at subfreezing temperatures. Thus, for example, at about 10°F. to −10°F. (Ca. −12°C. to −23°C.) the solvent rapidly becomes rather viscous. The increased viscosity tends to reduce the rate of solvent flow through a gas absorber so that less and less gas can be treated. Further at this low temperature the solvent starts to freeze, fouling heat exchangers. Then mass transfer problems begin to appear further reducing the efficiency of the gas treating operation. The solvent according to the present invention markedly alleviates and in some instances completely eliminates these problems.

The improved solvent composition according to the present invention comprises a mixture of dimethyl ethers of polyethylene glycols of the formula:

$$CH_3O(C_2H_4O)_xCH_3 \qquad (I)$$

wherein x is from 2 to 8. The mixture has an average molecular weight of from 200 to 255, and preferably from 227 to 244, and the homolog distribution (I) is as follows:

| x | Molecular homolog wt. % |
|---|---|
| 2 | 0.1—1 |
| 3 | 8—50 |
| 4 | 28—40 |
| 5 | 7—33 |
| 6 | 2—24 |
| 7 | 0—5 |
| 8 | 0—2 |

A preferred homolog distribution is as follows:

$$CH_3O(C_2H_4O)_xCH_3 \qquad (II)$$

| x | Molecular homolog .wt. % |
|---|---|
| 2 | 0.1—0.5 |
| 3 | 12—22 |
| 4 | 30—36 |
| 5 | 28—33 |
| 6 | 8—12 |
| 7 | 2—5 |
| 8 | 0.1—1.0 |

The above solvent composition II has a vapor pressure of about 0.0003 mm of Hg (0.0041 kg/m$^2$) and a viscosity of about 11 centipoises at a temperature of about 15°F (−9.4°C) and a vapor pressure of about 0.0001 mm of Hg (0.0013 kg/m$^2$) and a viscosity of about 18 centipoises at a temperature of about 0°F (−18°C).

The solvent composition may be used in known processes for separating hydrogen sulfide, carbon dioxide and mercaptans from gases, and it is particularly suitable in a subfreezing environment. As an example, the instant solvent composition may be used in place of the solvent SELEXOL disclosed and described in the aforementioned U.S. Patent 3,737,392. The disclosure in the 3,737,392 patent for treating gases is accordingly incorporated herein by reference. Another example would be the process disclosed in U.S. Patent 3,362,133.

The mixture of dimethyl ethers of polyethylene glycols of the present invention is prepared from the

corresponding monomethyl ethers of polyethylene glycols by reacting the monomethyl ether, with sodium to form the sodium alcoholate thereof, reacting the sodium alcoholate with methyl chloride to form the dimethyl ethers and sodium chloride, and separating the sodium chloride from the dimethyl ethers. Preferably, about 0.1—0.6 weight per cent of water is incorporated in the monomethyl ethers, and the reaction temperature is maintained at about 100—120°C.

The mixture of monomethyl ethers of polyethylene glycols from which the diethers are derived may be prepared from ethylene oxide and methanol. About 4.5 mols of ethylene oxide is reacted with 1 mol of methanol at about 110—140°C using sodium hydroxide as catalyst. The resulting product is distilled at about 10 mm Hg (136 kg/m$^2$) pressure to remove all of the low-boiling compounds, together with most of the monomethyl ether of triethylene glycol.

The most preferred solvent composition has the following approximate homolog distribution:

$$CH_3O(C_2H_4O)_xCH_3 \qquad (III)$$

| x | Molecular homolog, wt. % |
|---|---|
| 2 | 0.5 |
| 3 | 17.0 |
| 4 | 33.0 |
| 5 | 33.0 |
| 6 | 10.5 |
| 7 | 5.0 |
| 8 | 1 |

m. wt.=237.0

An example of the important physical properties of the most preferred solvent composition III are listed below:

| | |
|---|---|
| Vapor pressure, 15°F (−9.4°C), mm Hg | 0.0003 (0.0041 kg/m$^2$) |
| Viscosity, 15°F (−9.4°C), cp. | 11 |
| Viscosity, 50°F (10°C), cp. | 5.5 |
| Viscosity, 90°F (32°C), cp. | 3.3 |
| Specific heat, 41°F (5°C), | 0.46 |
| Freezing point, °F, | −20 to −36 (−29°C to −38°C) |
| Pounds per gallon, 77°F (25°C), | 8.5 (1.01 g/cm$^3$) |
| Flash point, °F, (COC) | 300 (149°C) |

It is of course understood that in the above formulations (I, II or III), minor amounts of lower and higher homologs may be tolerated so long as the physical properties which will permit the solvent to be efficient at sub-freezing temperatures e.g. viscosity are not affected.

A preferred process for removing acid gas from a gas mixture containing acidic components e.g. carbon dioxide, hydrogen sulfide and mercaptans may be practiced according to the following steps:

(a) contacting said gaseous mixture in a first absorption zone under superatmospheric pressure with a solvent comprising a mixture of dimethyl ethers of polyethylene glycols according to II or III above preferably having dissolved therein at least 1 weight percent acid gas to effect absorption of most of the acid gas so as to partially purify the gas;

(b) contacting said partially purified gas from the first absorption zone in a second absorption zone under superatmospheric pressure with the same solvent containing less than 1 weight percent acid gas to produce a purified gas;

(c) passing the solvent containing dissolved gases from the second absorption zone to the first absorption zone;

(d) passing the solvent containing dissolved acid gas from the first absorption zone to a flashing zone maintained at a pressure substantially lower than that in the first absorption zone to effect liberation of most of the acid gas therefrom;

(e) withdrawing from said flashing zone gases liberated therein;

(f) passing a major portion of solvent containing at least 1 weight percent acid gas from said flashing zone to said first absorption zone;

(g) passing a minor portion of solvent containing at least 1 weight percent acid gas from said flashing zone to a stripping zone;

(h) stripping substantially all of the acid gas from said minor portion of solvent; and

(i) returning desorbed solvent to the second absorption zone for further contact with the gaseous mixture.

In accordance with claim 5 of the invention steps (a) and (b) are carried out at subfreezing temperature.

Example I

Preparation of a mixture of monomethyl ethers of polyethylene glycols

A carbon steel reactor equipped with a central agitator and means for heating and cooling was charged with 7 lbs. (3.1 kg) of 50 weight percent aqueous sodium hydroxide and 865 lbs. (390 kg) of methanol. The mixture was heated to 110°C, and 4757 lbs. (2140 kg) of ethylene oxide was added to the mixture over a period of several hours. Reaction temperature was maintained at 110—140°C by cooling. The pressure remained below 150 p.s.i.a. (105 kg/m$^2$). The resulting mixture was then cooled to 60°C and stored. This crude product had an average molecular weight of 204 and contained monomethyl ether of mono-through octaethylene glycols.

The crude product was batch distilled in a 4 plate column operating at a pressure of 10 mm Hg (136 kg/m$^2$) and a reflux ratio of 1. Lights were removed until the overhead temperature of the column reached about 155°C. The column bottoms were taken as product and had the following composition:

| Monomethyl ether of: | Weight percent |
|---|---|
| Triethylene glycol | 17 |
| Tetraethylene glycol | 33 |
| Pentaethylene | 33 |
| Hexaethylene glycol | 11 |
| Heptaethylene glycol | 5 |
| Octaethylene glycol | 1 |

Average molecular weight of this mixture of monomethyl ethers of polyethylene glycols was about 224.

Example II

Preparation of a mixture of dimethyl ethers of polyethylene glycols

A stainless steel reactor equipped with means for agitating, heating, and cooling was charged with 1080 lbs. (486 kg) of monomethyl ethers of polyethylene glycols prepared in Example I and having an average molecular weight of about 224. About 4.6 lbs. (2.1 kg) of water was added and the mixture was agitated and heated to 110°C. About 112 lbs. (50.4 kg) of molten sodium at 135—140°C, was added to the mixture over a period of several hours while the mixture was maintained at about 120—130°C, by cooling. Hydrogen formed during the reaction was vented. The reaction yielded the sodium alcoholate of the monomethyl ethers of polyethylene glycol.

Next 305 lbs. (137 kg) of methyl chloride was added to the reaction mixture over a period of several hours while the reaction mixture was maintained at about 115—125°C. by cooling. The resulting reaction yielded sodium chloride and the dimethyl ethers of polyethylene glycols. The sodium chloride was separated from the reaction mixture in a centrifuge, and the resulting liquid product was stripped with natural gas to remove any excess methyl chloride. The stripped product was filtered, allowed to stand 24 hours and refiltered to remove any residual sodium chloride. This product may be distilled, if desired, but distillation is not necessary. The undistilled product had the following compositions and an average molecular weight of 237:

| Dimethyl ether of: | Weight percent |
|---|---|
| Triethylene glycol | 17 |
| Tetraethylene glycol | 33 |
| Pentaethylene glycol | 33 |
| Hexaethylene glycol | 11 |
| Heptaethylene glycol | 5 |
| Octaethylene glycol | 1 |
| Water | 1 |

The properties of the undistilled, filtered product are shown below:

| | Filtered product |
|---|---|
| $CO_2$ solubility wt. percent; | |
| At 215 p.s.i.a. (151 Mg/m$^2$) partial pressure at 70°F (28°C) | 9.8 |
| At 900 p.s.i.a. (603 Mg/m$^2$) partial pressure at 70°F (28°C) | 19.3 |
| H2S solubility, wt. percent: | |
| At 39 p.s.i.a. (27.4 Mg/m$^3$) partial pressure at 70°F (28°C) | 8.7 |
| At 70 p.s.i.a. (49 Mg/m$^2$) partial pressure at 70°F (28°C) | 15.6 |
| Chlorides p.p.m. | 8 |
| Viscosity, centipoises at 82°F (21°C) | 3.7 |
| Density, lbs/gal. at 82°F (21°C) | 8.5 (1.02 g/cm$^3$) |

Example III (for comparison)
Preparation of a low molecular weight mixture of the dimethyl ethers of polyethylene glycol

A vacuum distillation boiler is charged with 1005 lbs. (455 kg) of the dimethyl ethers of polyethylene glycol prepared in Example II. The 10-plate column was operated at 10 mm Hg (136 kg/m$^2$) and a reflux ratio of 1. Overhead product was condensed and collected until the overhead temperature reached 170°C. About 1005 lbs. (455 kg) of condensed product was collected and the composition was as follows:

| Dimethyl ether of: | Wt. percent |
|---|---|
| Diethylene glycol | 1 |
| Triethylene glycol | 51 |
| Tetraethylene glycol | 45 |
| Pentaethylene glycol | 3 |
| Hexaethylene glycol | — |
| Heptaethylene glycol | — |
| Octaethylene glycol | — |

Solubilities of carbon dioxide and hydrogen sulfide in the distilled product were similar to those of the higher molecular weight, undistilled product.

**Claims**

1. A mixture of dimethyl ethers of polyethylene glycol of the formula $CH_3O(C_2H_4O)_xCH_3$ wherein x is 2 to 8, characterized by an average molecular weight of from 200 to 255 and a molecular homolog distribution in terms of x of:

| x | Molecular homolog weight percent |
|---|---|
| 2 | 0.1—1 |
| 3 | 8—50 |
| 4 | 28—40 |
| 5 | 7—33 |
| 6 | 2—24 |
| 7 | 0—5 |
| 8 | 0—2 |

2. A mixture of dimethyl ethers of polyethylene glycol according to claim 1, characterized in that the molecular homolog distribution in terms of x is:

| x | Molecular homolog weight percent |
|---|---|
| 2 | 0.1—0.5 |
| 3 | 12—22 |
| 4 | 30—36 |
| 5 | 28—33 |
| 6 | 8—12 |
| 7 | 2—5 |
| 8 | 0.1—1.0 |

and wherein when measured at 15°F. (−9.4°C) said mixture has a vapor pressure of about 0.0003 mm of Hg (0.0041 kg/m$^2$) and a viscosity of about 11 centipoises.

3. A mixture according to claim 2, characterized in that x is 2 to 8 and the molecular homolog distribution in terms of x is about:

| x | Molecular homolog weight percent |
|---|---|
| 2 | 0.5 |
| 3 | 17 |
| 4 | 33 |
| 5 | 33 |
| 6 | 10.5 |
| 7 | 5.0 |
| 8 | 1 |

4. A mixture according to any one of claims 1 to 3, characterized in that the average molecular weight is between 227 and 244.

5. A process for separating at least one acid gas from a mixture of at least one acid gas with at least one other gas, comprising:

(a) contacting said gaseous mixture in a first absorption zone under superatmospheric pressure with a solvent comprising a mixture of dimethyl ethers of polyethylene glycols of the formula $CH_3O(C_2H_4O)_xCH_3$, wherein x is an integer, to effect absorption of most of the acid gas so as to partially purify the gaseous mixture;

(b) contacting said partially purified gaseous mixture from the first absorption zone in a second absorption zone under superatmospheric pressure with the same solvent containing less than 1% acid gas to produce a purified gas mixture;

(c) passing the solvent containing dissolved acid gas from the second absorption zone to the first absorption zone;

(d) passing the solvent containing dissolved acid gas from the first absorption zone to a flashing zone maintained at a pressure substantially lower than that in the first absorption zone to effect liberation of most of the acid gas therefrom;

(e) withdrawing from said flashing zone any gases liberated therein;

(f) passing a major portion of solvent containing at least 1 weight percent acid gas from said flashing zone to said first absorption zone;

(g) passing a minor portion of solvent containing at least 1 weight percent acid gas from said flashing zone to a stripping zone;

(h) stripping substantially all of the acid gas from said minor portion of solvent; and

(i) returning desorbed solvent to the second absorption zone for further contact with the gaseous mixture, characterized by the fact that in step (a) as the mixture of dimethyl ethers of polyethylene glycols a mixture as defined in claims 2 or 3, preferably having an average molecular weight as defined in claim 4, is used and that steps (a) and (b) are carried out at a subfreezing temperature.

**Patentansprüche**

1. Gemisch von Dimethylethern des Polyethylenglykols der Formel $CH_3O(C_2H_4O)_xCH_3$, in der x gleich 2 bis 8 ist, gekennzeichnet durch ein durchschnittliches Molekulargewicht von 200 bis 255 und durch folgende Verteilung der durch den Wert von x bezeichneten Molekularhomologe:

| x | Molekularhomolog Gew.% |
|---|---|
| 2 | 0,1 bis 1 |
| 3 | 8 bis 50 |
| 4 | 28 bis 40 |
| 5 | 7 bis 33 |
| 6 | 2 bis 24 |
| 7 | 0 bis 5 |
| 8 | 0 bis 2 |

2. Gemisch von Dimethylethern des Polyethylenglykols nach Anspruch 1, dadurch gekennzeichnet, daß es folgends Verteilung der durch den Wert von x bezeichneten Molekularhomologe hat:

| x | Molekularhomolog Gew.% |
|---|---|
| 2 | 0,1 bis 0,5 |
| 3 | 12 bis 22 |
| 4 | 30 bis 36 |
| 5 | 28 bis 33 |
| 6 | 8 bis 12 |
| 7 | 2 bis 5 |
| 8 | 0,1 bis 1,0 |

und daß bei einer Messung bei −9,4°C das Gemisch einen Dampfdruck von etwa 0,0041 kg/m$^2$ und eine Viskosität von etwa 11 mPa s hat.

3. Gemisch nach Anspruch 2, dadurch gekennzeichnet, daß x gleich 2 bis 8 ist und daß es folgende Verteilung der durch den Wert von x bezeichneten Molekularhomologe hat:

| x | Molekularhomolg Gew.% |
|---|---|
| 2 | 0,5 |
| 3 | 17 |
| 4 | 33 |
| 5 | 33 |
| 6 | 10,5 |
| 7 | 5,0 |
| 8 | 1 |

4. Gemisch nach einem der Ansprüche 1 bis 3, dadurch gekennzeichnet, daß das durchschnittliche Molekulargewicht zwischen 227 und 244 liegt.

5. Verfahren zum Abtrennen mindestens eines Sauergases von einem Gemisch aus mindestens einer säurebildenden Gaskomponente mit mindestens einem anderen Gas, in dem

(a) das Gasgemisch in einer ersten Absorptionszone unter einem Überdruck mit einem Lösungsmittel in Berührung gebracht wird, das mindestens teilweise aus einem Gemisch von Dimethylethern von Polyethylenglykolen der Formel $CH_3O(C_2H_4O)_xCH_3$ besteht, in der x eine ganze Zahl ist, so daß das Gasgemisch durch die Absorption des größten Teils des Sauergases teilgereinigt wird;

(b) das von der ersten Absorptionszone kommende, teilgereinigte Gasgemisch in einer zweiten Absorptionszone unter einem Überdruck mit demselben Lösungsmittel in Berührung gebracht wird, das weniger als 1% säurebildenden Gasanteil enthält und dadurch ein gereinigtes Gasgemisch erzeugt wird;

(c) einen gelösten säurebildenden Gasanteil enthaltendes Lösungsmittel aus der zweiten Absorptionszone in die erste Absorptionszone geleitet wird,

(d) einen gelösten säurebildenden Gasanteil enthaltendes Lösungsmittel aus der ersten Absorptionszone in eine Entspannungsverdampfungszone eingeleitet wird, in der ein beträchtlich niedrigerer Druck als in der ersten Absorptionszone aufrechterhalten und dadurch der größte Teil des säurebildenden Gasanteils aus dem Lösungsmittel freigesetzt wird;

(e) von der Entspannungsverdampfungszone in ihr freigesetzte Gase abgezogen werden;

(f) von dem mindestens 1 Gew.% saure Gasanteil enthaltenden Lösungsmittel von der Entspannungsverdampfungszone der größte Teil in die erste Absorptionszone eingeleitet wird;

(g) von dem mindestens 1 Gew.% säurebildenden Gasanteil enthaltenden Lösungsmittel von der Entspannungsverdampfungszone ein kleinerer Teil in eine Abstreifzone eingeleitet wird;

(h) in der Strippzone der säurebildende Gasanteil im wesentlichen vollständig abgestreift wird; und

(i) desorbiertes Lösungsmittel in der zweiten Absorptionszone erneut mit dem Gasgemisch in Berührung gebracht;

dadurch gekennzeichnet, daß im Schritt (a) als das Gemisch von Dimethylethern von Polyethylenglykolen ein Gemisch nach Anspruch 2 oder 3, vorzugsweise mit einem durchschnittlichen Molekulargewicht nach Anspruch 4, verwendet wird und daß die Schritte (a) und (b) bei einer Minustemperatur durgeführt werden.

**Revendications**

1. Mélange d'éthers diméthyliques de polyéthylène-glycol de formule:

$$CH_3O(C_2H_4O)_xCH_3$$

dans laquelle x est un nombre de 2 à 8, caractérisé par une masse moléculaire moyenne de 200 à 255 et une distribution d'homologues moléculaires exprimée en x de:

| x | Homologues moléculaires % en poids |
|---|---|
| 2 | 0,1—1 |
| 3 | 8—50 |
| 4 | 28—40 |
| 5 | 7—33 |
| 6 | 2—24 |
| 7 | 0—5 |
| 8 | 0—2 |

2. Mélange d'éthers diméthyliques de polyéthylène-glycol selon la revendication 1, caractérisé en ce que la distribution des homologues moléculaires exprimée par x est:

| x | Homologues moléculaires % en poids |
|---|---|
| 2 | 0,1—0,5 |
| 3 | 12—22 |
| 4 | 30—36 |
| 5 | 28—33 |
| 6 | 8—12 |
| 7 | 2—5 |
| 8 | 0,1—1,0 |

et dans lequel, quand on la mesure à −9,4°C (15°F) la tension de vapeur de ce mélange est d'environ 0,0003 mm de Hg (0,0041 kg/$m^2$) et la viscosité est d'environ 11 cp.

3. Mélange selon la revendication 2, caractérisé en ce que x est de 2 à 8 et la distribution des homologues moléculaires exprimée par x est d'environ:

| x | Homologues moléculaires % en poids |
|---|---|
| 2 | 0,5 |
| 3 | 17 |
| 4 | 33 |
| 5 | 33 |
| 6 | 10,5 |
| 7 | 5,0 |
| 8 | 1 |

4. Mélange selon l'une quelconque des revendications 1 à 3, caractérisé en ce que la masse moléculaire moyenne est de 227 à 244.

5. Procédé pour séparer au moins un gaz acide d'un mélange d'au moins un gaz acide avec au moins un autre gaz, consistant à:

(a) mettre en contact ledit mélange gazeux dans une première zone d'absorption sous une pression supérieure à la pression atmosphérique avec un solvant comprenant un mélange d'éthers diméthyliques de polyéthylèneglycols de formule:

$$CH_3O(C_2H_4O)_xCH_3$$

dans laquelle x est un nombre entier, pour effectuer l'absorption de la majeure partie du gaz acide afin de purifier partiellement le mélange gazeux;

(b) mettre en contact ledit gaz partiellement purifié provenant de la première zone d'absorption dans une seconde zone d'absorption, sous une pression supérieure à la pression atmosphérique avec le même solvant contenant moins de 1% en poids de gaz acide pour obtenir un gaz purifié;

(c) faire passer le solvant contenant les gaz dissous de la seconde zone d'absorption à la première zone d'absorption;

(d) faire passer le solvant contenant le gaz acide dissous de la première zone d'absorption à une zone de détente maintenue sous une pression sensiblement inférieure à celle dans la première zone d'absorption afin d'effectuer la libération de la majeure partie du gaz acide de celui-ci;

(e) soutirer de ladite zone de détente les gaz libérés dans celle-ci;

(f) faire passer la majeure partie du solvant contenant au moins 1% en poids de gaz acide de ladite zone de détente à ladite première zone d'absorption;

(g) faire passer une petite proportion de solvant contenant au moins 1% en poids de gaz acide de ladite zone de détente à une zone de distillation;

(h) distiller sensiblement la totalité du gaz acide de ladite petite proportion de solvant; et

(i) recycler le solvant désorbé à la seconde zone d'absorption en vue d'un nouveau contact avec le mélange gazeux,

caractérisé en ce qu'au stade (a) on utilise à titre de mélange d'éthers diméthyliques de polyéthylène-glycols, un mélange tel que défini dans la revendication 2 ou 3 ayant de préférence une masse moléculaire moyenne telle que définie dans la revendication 4, et en ce que les stades (a) et (b) sont effectués à une température de sous-réfrigération.